# FASCICULE DE BREVET EUROPEEN

(11) **EP 0 906 443 B1**
(45) Date de publication et mention de la délivrance du brevet: **10.08.2005**
(21) Numéro de dépôt: 97925133.7
(22) Date de dépôt: 23.05.1997
(51) Int. Cl.: C12N 15/86, C12N 5/10, C12N 15/10, C12N 15/85, A61K 48/00

(54) **GENERATION DE MOLECULES REPLICATIVES IN VIVO**
IN VIVO HERSTELLUNG VON REPLICATIVEN MOLEKÜLEN
GENERATING REPLICATING MOLECULES IN VIVO

(30) Priorité: 12.06.1996 FR 9607273
(43) Date de publication de la demande: 07.04.1999
(73) Titulaire: GENCELL S.A., 94400 Vitry sur Seine (FR)
(72) Inventeur: PERRICAUDET, Michel, F-28320 Ecrosnes (FR); YEH, Patrice, F-75005 Paris (FR); LEBLOIS-PREHAUD, Hélène, F-78280 Guyancourt (FR)
(74) Mandataire: Lecca, Patricia
(86) Numéro de dépôt international: PCT/FR1997/000914
(87) Numéro de publication internationale: WO 1997/047757

(56) Documents cités:
- EP-A- 0 704 534
- WO-A-94/29438
- PROCEEDINGS OF THE NATIONAL ACADEMY OF SCIENCES OF USA, vol. 92, no. 13, Juin 1995, WASHINGTON US, pages 5940-5944, XP002025745 LOGIE, C. ET STEWART A. F.: "Ligand-regulated site specific recombination"
- SOMATIC CELL AND MOLECULAR GENETICS, vol. 21, no. 6, Novembre 1995, pages 429-441, XP000617918 WANG, P. ET AL.: "High frequency recombination between loxP sites in human chromosomes mediated by an adenovirus vector expressing Cre recombinase"
- BIOTECHNOLOGY AND BIOENGINEERING INCLUDING: SYMPOSIUM BIOTECHNOLOGY IN ENERGY PRODUCTION AND CONSERVATION., vol. 217, no. 2, 14 Décembre 1995, NEW YORK US, pages 393-401, XP000644451 SAKAI, K. ET AL: "Efficient regulation of gene expression by adenovirus vector-mediated delivery of cre recombinase"
- NUCLEIC ACIDS RESEARCH, vol. 23, no. 21, 11 Novembre 1995, OXFORD GB, pages 4451-4456, XP000644463 BERGEMANN, J. ET AL.: "Excision of specific DNA-sequences from integrated retroviral vectors via site-specific recombination"
- PROCEEDINGS OF THE NATIONAL ACADEMY OF SCIENCES OF USA, vol. 93, no. 9, Avril 1996, WASHINGTON US, pages 3932-3936, XP000644460 WANG, Y. ET AL.: "Targeted DNA recombination in vivo using an adenovirus carrying the cre recombinase gene"

## Description

La présente invention concerne des molécules d'ADN circulaires et réplicatives, utilisables en thérapie génique. L'invention décrit également une méthode particulièrement efficace pour leur génération in situ à partir d'un vecteur viral correspondant.

La thérapie génique consiste à corriger une déficience ou une anomalie (mutation, expression aberrante, etc.) par introduction d'une information génétique dans la cellule ou l'organe affecté.

Cette information génétique peut être introduite soit in vitro dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des vecteurs de diverses natures. Il peut s'agir de vecteurs chimiques et/ou biochimiques, naturels ou synthétiques d'une part ou de vecteurs viraux d'autre part. A titre illustratif des vecteurs viraux, on peut notamment citer les complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), les liposomes (Fraley et al., J.Biol.Chem. 255 (1980) 10431), etc. Toutefois, leur utilisation implique notamment la possibilité de produire des quantités importantes d'ADN de pureté pharmacologique.

Les vecteurs viraux (rétrovirus, adénovirus, virus adéno-associés,...) sont très efficaces, comparativement aux vecteurs chimiques et/ou biochimiques précédemmment décrits, notamment pour le passage des membranes. Parmi ces virus, les adénovirus présentent tout particulièrement, des propriétés intéressantes pour une utilisation en thérapie génique. Notamment, ils ont un spectre d'hôte assez large, sont capables de transduire des cellules en division ou bien des cellules quiescentes et le génome de l'adenovirus persiste sous forme extrachromosomale, de plus ils n'ont pas été associés à ce jour à des pathologies importantes chez l'homme. En revanche, l'utilisation du retrovirus dont le génome s'intègre de façon aléatoire dans le génome de la cellule infectée est limité aux cellules en division. Les adénovirus ont ainsi été utilisés pour transférer des gènes d'intérêt dans des cellules quiescentes musculaires (myotubes; Ragot et al., Nature 361 (1993) 647)), hépatiques (Jaffe et al., Nature genetics 1 (1992) 372), nerveuses (Akli et al., Nature genetics 3 (1993) 224), épithéliales bronchiques (Rosenfeld et al., 1992), etc. Cependant dans les tissus à renouvellement rapide on observe une perte progressive de l'expression du transgène par effet de dilution au cours des divisions cellullaires.

L'amélioration des vecteurs adénoviraux et le développement de nouvelles générations de vecteurs a porté sur la diminution des risques potentiels de pouvoir pathogène résiduels ainsi que de pouvoir immunogène liés à la réplication du vecteur, la recombinaison de son génome et l'expression de protéines virales.

Pour prévenir au maximum de tels risques, les constructions de vecteurs viraux actuellement proposées sont modifiées de manière à rendre lesdits vecteurs incapables de se répliquer de façon autonome dans la cellule cible. Ils sont dits défectifs. Généralement, le génome des virus défectifs est donc dépourvu au moins des séquences nécessaires à la réplication dudit virus dans la cellule infectée. Ainsi dans le cas particulier des adénovirus, les constructions décrites dans l'art antérieur sont des adénovirus délétés des régions E1 et éventuellement E3 au niveau desquelles sont insérées les séquences d'ADN hétérologues (Levrero et al., Gene 101 (1991) 195; Gosh-Choudhury et al., Gene 50 (1986) 161). D'autres constructions comportent une délétion au niveau de la région E1 et d'une partie non essentielle de la région E4 (WO94/12649), ou une organisation génomique modifiée (FR 94 13355).

Néanmoins, il demeure, lors de la production de ces vecteurs viraux défectifs, le risque de recombinaisons générant des particules virales réplicatives ou des transcomplémentations in vivo par des fonctions cellulaires de type E1. Il est clair que l'utilisation en thérapie génique, de vecteurs ainsi contaminés, peut avoir des conséquences très néfastes comme par exemple induire une propagation virale et provoquer une dissémination incontrôlée avec des risques de réaction inflammatoire et de réponse immunitaire dirigée contre les protéines virales, etc...

D'autre part, l'amélioration, de la stabilité de l'expression du transgène dans les cellules transduites par le vecteur adénoviral, et plus particulièrement dans les cellules en division reste un problème important à résoudre. En conséquence, il demeure à ce jour en thérapie génique, un besoin réel en vecteurs de transfert qui manifesteraient essentiellement les avantages de chacun des vecteurs précédemment décrits à savoir de bonnes propriétés de transfection, calquées par exemple sur celles des vecteurs viraux et en particulier celles des adénovirus et une parfaite innocuité se traduisant en particulier par une absence de génération de particules virales réplicatives in vivo, risque inexistant avec les plasmides ou vecteurs non viraux.

A ce sujet, la demande européenne EP 704 534 décrit un vecteur adénoviral recombinant pour le transfert d'un transgene dans des cellules animales hôtes, le vecteur adénoviral utilisé étant capable de se répliquer de manière autonome dans la cellule animale transfectée présente une meilleure stabilité de l'expression du transgène. Plus particulièrement, il est décrit un vecteur adénoviral défectif comprenant entre deux séquences permettant une recombinaison site-spécifique, une origine de réplication telle que par exemple l'origine de réplication de SV40, un promoteur, un gène hétérologue et une séquence de polyadénylation. Ce vecteur adénoviral est maintenu de manière stable dans les cellules animales hôtes et le transgène est activé et s'exprime indépendamment de l'événement de recombinaison site-spécifique. Bergemann J et al. (Nucleic Acids Res. 1995 Nov 11;23(21):4451-6) décrit un système dit retroviral lox pour le transfert et l'expression de transgenes dans les cellules transfectées. Le vecteur rétroviral comprend notamment une cassette d'ADN excisable codant pour un transgene d'intérêt entouré de deux sites loxP permettant dans un premier temps l'infection et l'intégration du vecteur, et l'expression de manière stable du gène ainsi transféré et dans un deuxième temps une inhibition du transgene par un événement d'excision spécifique en présence de l'enzyme de recombinaison Cre. Alternativement, Logie et C. (PNAS, vol. 92, pp. 5940-5944, Juin 1995) décrit l'utilisation d'enzyme de recombinaison inductible.

L'objectif de la présente invention est précisément de proposer un nouveau concept de transfert de gènes satisfaisant aux exigences précitées. La présente invention réside notamment dans la génération in situ, via un vecteur viral, de molécules d'ADN circulaires, réplicatives, thérapeutiques et avantageuses sur le plan de la stabilité de l'expression du transgène et de l'innocuité. En effet, celles-ci sont dépourvues de toute séquence du génôme viral susceptible d'induire une réponse immunitaire de type inflammatoire ainsi qu'une réponse spécifique dirigée contre les protéines virales pouvant avoir un effet délétère sur l'organisme et limiter la durée d'expression du transgène.

La présente invention a ainsi pour objet un vecteur viral comprenant une région d'ADN délimitée par deux séquences permettant une recombinaison site spécifique et positionnées en orientation directe, ladite région comprenant successivement un promoteur fonctionnel dans les cellules mammifères, la séquence oriP du virus EBV, une cassette d'expression comportant un gène d'intérêt et le gène de la protéine EBNA1 séparés par une séquence IRES dérivée du virus ECMV, le promoteur et la cassette d'expression étant orientés de telle sorte que l'expression des deux gènes n'est possible qu'après la recombinaison site spécifique.

La présente invention découle en particulier de la mise au point d'un procédé et de constructions spécifiques, particulièrement efficaces pour la production de ces molécules d'ADN thérapeutiques. Plus particulièrement, le procédé selon l'invention réside dans la production des molécules d'ADN thérapeutiques définies ci-avant à partir d'un vecteur viral.

De manière inattendue, la demanderesse a ainsi mis en évidence qu'il était possible de générer in situ à partir d'un vecteur viral et ceci par recombinaison site spécifique, une molécule d'ADN circulaire, à caractère thérapeutique et réplicative. De plus, dans un mode de réalisation avantageux, le transgène et l'origine de réplication sont inactifs dans le vecteur viral, leur activité étant dépendante de l'événement de recombinaison site spécifique. Encore plus avantageusement, l'événement de recombinaison est induit de façon conditionnelle par l'expression de la recombinase, ce qui offre un grand niveau de contrôle sur l'expression du gène d'intérêt et sur la réplication des molécules épisomiques produites.

Un tel protocole est particulièrement avantageux sur le plan thérapeutique:
- il tire partie des bonnes capacités de transfection manifestées de manière générale par les vecteurs viraux comparativement aux vecteurs non viraux,
- il réduit considérablement les risques de contamination virale, de réaction inflammatoire locale ainsi que de réponse immunitaire antivirale, compte tenu de la faible quantité de vecteurs viraux mis en oeuvre. Ce dernier n'est introduit que dans une proportion nécessaire à la génération de la molécule d'ADN thérapeutique,
- il permet d'élargir le domaine d'application de certains vecteurs viraux : Ainsi, les vecteurs adénoviraux sont d'applications limitées dans les cellules prolifératives telles que les cellules souches hématopoïétiques. La présente invention permet d'exploiter leur pouvoir infectieux pour générer dans des cellules prolifératives des molécules circulaires réplicatives stables.

La molécule d'ADN telle que revendiquée, possède donc la faculté d'assurer efficacement le transfert dans les cellules visées du ou des gène(s) thérapeutiques qu'elle contient.

Pour ce faire elle contient une origine de réplication caractérisée notamment par le fait qu'elle est fonctionnelle dans des cellules de mammifères et humaines. Avantageusement, l'origine de replication utilisée est une origine de replication conditionnelle, c'est-à- dire dont l'activité peut être régulée. De manière encore plus preférée, l'origine de replication est disposée de telle sorte qu'elle soit inactive dans le vecteur viral, et active après la recombinaison site-spécifique.

Avantageusement, l'origine de réplication utilisée est d'origine eucaryote, virale ou mammifère.

Un exemple préféré d'origine de réplication susceptible d' être mise en oeuvre dans le cadre de la présente invention est plus particulièrement l'origine de réplication du virus Epstein Barr (EBV). Le virus de l'EBV appartient à la famille des Herpesviridae. Son origine de replication comprend deux éléments : la séquence oriP (1,7kb) responsable de la replication, dont l'activite est induite par la protéine codee par le gène EBNA1. Cette sequence peut etre portee en trans. Ces éléments permettent à eux seuls à la fois la réplication, le maintien épisomal et à la ségrégation de 5 à 20 copies par cellule d'un vecteur plasmidique. La séquence oriP est composée d'une répétition de 20 motifs de 30bp, séparée de 960bp de l'origine de réplication qui est formée d'un motif inversé répété de 65bp et comprend 4 copies imparfaites du motif de 30 bp. La protéine EBNA1 se fixe sur les motifs de 30bp au niveau de l'origine de réplication et permet le recrutement de facteurs cellulaires au moment de la phase S et la réplication, synchrone à la division cellulaire, d'un plasmide possédant la séquence oriP en cis. De plus EBNA1, vraisemblablement par la fixation simultanée au niveau des motifs répétés et de structures chromosomales, permet le maintien intranucléaire et la ségrégation de l'épisome au moment de la divison cellulaire. Des plasmides contenant l'origine de réplication oriP du génome d'EBV et permettant l'expression de la protéine virale EBNA1 (641 acides aminés) sont maintenus de façon épisomique stable dans les cellules humaines transfectées et leur réplication est synchrone à la division cellulaire (Lupton et Levine, 1985).

L'origine de réplication peut également être dérivée des papillomavirus. Les papillomavirus utilisent un système de latence virale avec maintien episomique du genome analogue a celui du virus Epstein Barr (EBV). Ce systeme a particulierement ete etudie pour le papillomavirus bovin de type 1 (BPV-1). L'origine de replication de BPV est active en présence des proteines E1 et E2. Comme dans le cas d'EBV. le maintien épisomal est indépendant de la replication et est assuré par la fixation d'E2 au niveau de la sequence MME (minichromosome maintenance élément), mais necessite aussi la presence d'E1. En revanche, contrairement au systeme oriP/EBNA1 d'EBV, la replication de l'épisome n'est pas synchrone à la division cellulaire (Piirsoo et coll., 1996).

L'origine de réplication peut encore être constituée de séquences capables de replication autonome, ou ARS (autonomously replicating sequences). Des ARS ont ete isolées à partir de chromosomes de mammifères, notamment l'homme et la souris. A titre préférentiel, on peut citer la séquence ARS localisée en amont du locus c-myc chez l'homme (Ariga et al., 1988) et le fragment de 4kb du locus du gène de l'adenosine deaminase de la souris (Virta-Pearlman et al., 1993).

En ce qui concerne le gène d'intérêt, il peut s'agir d'un gène thérapeutique, vaccinal, agronomique ou vétérinaire. Il contient également une région promotrice de la transcription fonctionnelle dans la cellule ou l'organisme cible, ainsi qu'une région située en 3', et qui spécifie un signal de fin de transcription et de polyadénylation. Concernant la région promotrice, il peut s'agir d'une région promotrice naturellement responsable de l'expression du gène considéré lorsque celle-ci est susceptible de fonctionner dans la cellule ou l'organisme concernés. Il peut également s'agir de régions d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule cible. Parmi les promoteurs eucaryotes, on peut utiliser tout promoteur ou séquence dérivée stimulant ou réprimant la transcription d'un gène de façon spécifique ou non, inductible ou non, forte ou faible. Il peut s'agir en particulier de promoteurs ubiquitaires (promoteur des gènes HPRT, PGK, a-actine, tubuline, etc), de promoteurs des filaments intermédiaires (promoteur des gènes GFAP, desmine, vimentine, neurofilaments, kératine, etc), de promoteurs de gènes thérapeutiques (par exemple le promoteur des gènes MDR, CFTR, Facteur VIII, ApoAI, etc), de promoteurs spécifiques de tissus (promoteur du gène pyruvate kinase, villine, protéine intestinale de liaison des acides gras, alpha-actine du muscle lisse, etc) ou encore de promoteurs répondant à un stimulus (récepteur des hormones stéroïdes, récepteur de l'acide rétinoïque, etc). De même, il peut s'agir de séquences promotrices issues du génome d'un virus, tel que par exemple les promoteurs des gènes EIA et MLP d'adénovirus, le promoteur précoce du CMV, ou encore le promoteur du LTR du RSV, etc. En outre, ces régions promotrices peuvent être modifiées par addition de séquences d'activation, de régulation, ou permettant une expression tissu-spécifique ou majoritaire. Par ailleurs, le gène d'intérêt peut également comporter une séquence signal dirigeant le produit synthétisé dans les voies de sécrétion de la cellule cible. Cette séquence signal peut être la séquence signal naturelle du produit synthétisé, mais il peut également s'agir de toute autre séquence signal fonctionnelle, ou d'une séquence signal artificielle.

On utilise avantageusement un promoteur d'origine virale choisi parmi le promoteur precoce du CMV ou le LTR d'un retrovirus ou un promoteur mammifere.

Outre une origine de réplication et au moins un gène d'intérêt, les vecteurs viraux de l'invention comprennent une région délimitée par deux séquences permettant une recombinaison site-spécifique et positionnées en orientation directe. Cette recombinaison site-spécifique peut être obtenue à partir de divers systèmes qui entraînent la recombinaison site-spécifique entre des séquences.

Le système de recombinaison spécifique mis en oeuvre dans le cadre de la présente invention en vue de la génération in situ des molécules d'ADN peut être de différentes origines. En particulier, les séquences spécifiques et les recombinases utilisées peuvent appartenir à différentes classes structurales, et notamment à la famille de la recombinase du bactériophageP1.

Plus préférentiellement, la recombinaison site-spécifique utilisée selon le procédé de l'invention est obtenue au moyen de deux séquences spécifiques qui sont capables de recombiner entre elles en présence d'une protéine spécifique, généralement désignée recombinase. C'est pour cette raison que les molécules d'ADN circulaires selon l'invention comprennent en outre une séquence résultant de cette recombinaison site spécifique. Les séquences permettant la recombinaison utilisées dans le cadre de l'invention comprennent généralement de 5 à 100 paires de bases, et, plus préférentiellement, moins de 50 paires de bases.

Parmi les recombinases appartenant à la famille de l'intégrase du bactériophage 1, on peut citer notamment l'intégrase des phages lambda (Landy et al., Science 197 (1977) 1147), P22 et F80 (Leong et al., J. Biol. Chem. 260 (1985) 4468), HP1 de Haemophilus influenzae (Hauser et al., J. Biol. Chem. 267 (1992) 6859), l'intégrase Cre du phage P1, l'intégrase du plasmide pSAM2 (EP 350 341) ou encore la FLP recombinase du plasmide 2 m de la levure Saccharomyces cerevisiae. Lorsque les molécules d'ADN selon l'invention sont préparées par recombinaison au moyen d'un système site spécifique de la famille de l'intégrase du bactériophage lambda, les molécules d'ADN selon l'invention comprennent généralement en outre une séquence résultant de la recombinaison entre deux séquences d'attachement att du bactériophage ou plasmide correspondant.

Parmi les recombinases appartenant à la famille du transposon Tn3, on peut citer notamment la résolvase du transposon Tn3 ou des transposons gd, Tn21 et Tn522 (Stark et al., 1992) ; l'invertase Gin du bactériophage mu ou encore la résolvase de plasmides, telle que celle du fragment par de RP4 (Abert et al., Mol. Microbiol. 12 (1994) 131). Lorsque les molécules d'ADN selon l'invention sont préparées par recombinaison au moyen d'un système site spécifique de la famille du transposon Tn3, les molécules d'ADN selon l'invention comprennent généralement en outre une séquence résultant de la recombinaison entre deux séquences de reconnaissance de la résolvase du transposon considéré.

Selon un mode de réalisation préféré, dans les constructions génétiques de la présente invention, les séquences permettant la recombinaison site-spécifique sont dérivées d'un bactériophage. Plus préférentiellement, il s'agit des séquences d'attachement (séquences attP et attB) d'un bactériophage ou de séquences dérivées. Ces séquences sont capables de recombiner spécifiquement entre-elles en présence d'une recombinase désignée intégrase. Le terme séquence dérivée inclut les séquences obtenues par modification(s) des séquences d'attachement des bactériophages, conservant la capacité de recombiner spécifiquement en présence de la recombinase appropriée. Ainsi, il peut s'agir de fragments réduits de ces séquences ou au contraire étendus par addition d'autres séquences (sites de restriction, etc). II peut également s'agir de variants obtenus par mutation(s), notamment par mutation(s) ponctuelle(s). On désigne selon l'invention par séquences attP et attB d'un bactériophage ou d'un plasmide les séquences du système de recombinaison spécifique dudit bactériophage ou plasmide, c'est-à-dire la séquence attP présente dans ledite phage ou plasmide et la séquence attB chromosomique correspondante.

A titre d'exemples préférés, on peut citer notamment les séquences d'attachement des phages lambda, P22, F80, P1, HP1 de Haemophilus influenzae ou encore du plasmide pSAM2, ou 2 m.

Selon un mode de réalisation préféré de l'invention, les séquences permettant la recombinaison site-spécifique sont dérivées du système de recombinaison du phage P1. Ce phage P1 possède une recombinase du nom de Cre qui reconnaît spécifiquement une séquence nucléotidique de 34 paires de bases appelée site lox P. Cette séquence est composée de deux séquences palindromiques de 13pb séparées par une séquence conservée de 8pb.

Dans une variante particulière, l'invention concerne donc une molécule d'ADN circulaire et réplicative comprenant (a) une séquence issue de la recombinaison site spécifique entre deux régions loxP du bactériophage P1, au moins un gène d'intérêt et une origine de réplication fonctionnelle dans les cellules de mammifères et humaines et qui selon un mode préférentiel possède une fonctionnalité conditionnelle.

A cet égard, la présente invention fournit des constructions géniques particulières appropriées à la production des molécules d'ADN thérapeutiques définies ci-avant. Ces constructions géniques, ou ADN recombinants selon l'invention comprennent notamment le ou les gènes d'intérêt, l'origine de réplication et le gène de la protéine EBNA1 encadrés par les deux séquences permettant la recombinaison site-spécifique positionnées en orientation directe. Ces séquences peuvent être clonées sous forme de cassettes dans des plasmides bactériens. L'ADN plasmidique pouvant, dans un premier temps, être transfecté dans des cellules humaines afin de tester la fonctionnalité de ces séquences. Ces cassettes sont ensuite utilisées pour construire des vecteurs viraux possédant ces mêmes séquences intégrées dans leur génôme.

Comme indiqué précédemment, un autre aspect de la présente invention réside dans un procédé de production in situ de molécules d'ADN thérapeutiques définies ci-avant à partir d'un vecteur viral par recombinaison site-spécifique. L'emploi d'un tel vecteur permet avantageusement d'optimiser l'administration de la molécule d'ADN revendiquée dans les cellules à traiter.

A cet égard, la présente invention a également pour objet un vecteur viral comprenant, inséré dans son génome, au moins une région d'ADN encadrée par deux séquences permettant une recombinaison site-spécifique et positionnées en orientation directe, ladite région d'ADN comportant au moins une origine de réplication et un gène d'intérêt.

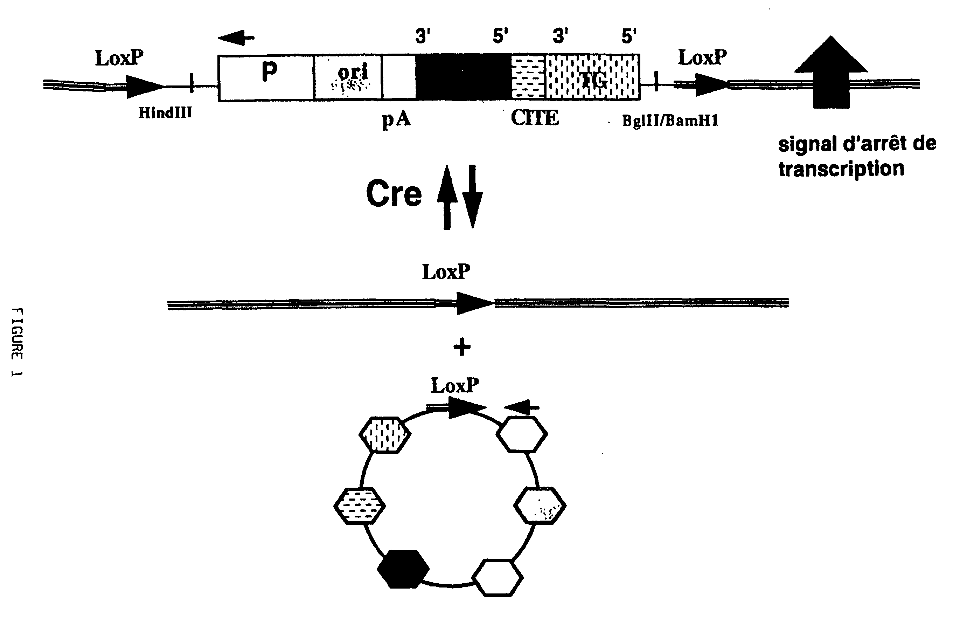

Selon un mode privilégié de l'invention, l'origine de réplication ainsi que le gène d'intérêt intégrés dans le vecteur viral sont présents sous une forme inactivée, le promoteur étant cloné en orientation directe à l'une des extrémités de la cassette d' expression (figure 1). Après recombinaison entre les deux sites LoxP le promoteur se retrouve devant le gène d'intérêt et séparé de ce dernier par un site LoxP, le premier ATG du transcrit correspondant au codon d'initiation du transgène. La séquence oriP n'est active qu'en présence de la protéine EBNA1 celle-ci est exprimée sous le contrôle du même promoteur que le transgène , sous forme d'un messager bicistronique. La traduction d'EBNA1 est initiée par un mécanisme d'initiation interne au niveau d'une séquence IRES dérivée du virus de l'encéphalomyocardite (ECMV), de la famille des picomavirus. L'expression du transgène et de la protéine EBNA1 ainsi que la réplication du plasmide sont donc directement conditionnés par l'évènement de recombinaison entre les deux sites LoxP.

Selon une autre variante de l'invention, la région d'encapsidation du virus est incluse dans le réplicon (la région d'ADN encadrée par les séquences permettant la recombinaison site-spécifique). Ce mode de mise en oeuvre offre une sécurité supplémentaire au système, comme expliqué plus loin.

Le vecteur viral utilisé peut être d'origines diverses, dès lors qu'il est capable de transduire les cellules animales et de préférence les cellules humaines. Dans un mode préféré de mise en oeuvre de l'invention, on utilise des vecteurs dérivés des adénovirus, des virus adéno-associés (AAV), des virus de l'herpès (HSV) ou des rétrovirus. Il est tout particulièrement avantageux d'utiliser un adénovirus, pour une administration directe ou pour la modification ex vivo de cellules destinées à être implantées, ou un rétrovirus, pour l'implantation de cellules productrices.

Les virus selon l'invention sont défectifs, c'est-à-dire qu'ils sont incapables de se répliquer de façon autonome dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence nucléique hétérologue d'intérêt. Préférentiellement, le virus défectif conserve néanmoins les séquences de son génome qui sont nécessaires à l'encapsidation des particules virales.

S'agissant plus particulièrement d'adénovirus, on préfère utiliser dans le cadre de la présente invention les adénovirus humains de type 2 ou 5 (Ad2 ou Ad5) ou les adénovirus d'origine animale (voir demande WO94/26914). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV).

Préférentiellement, les vecteurs viraux de l'invention sont des adénovirus défectifs comprenant inséré dans leur génome, une séquence génique comportant au moins une origine de réplication et un gène d'intérêt et encadrée par deux séquences positionnées en orientation directe. Celles-ci permettent d'induire une activité conditionnelle de l'origine de réplication et du transgène, par l'intermédiaire de la recombinaison site spécifique, dépendant de la présence de la recombinase.

Avantageusement, dans le génome de ces adénovirus de l'invention, la région E1 au moins est rendue non fonctionnelle. Encore plus préférentiellement le gène E1 et au moins l'un des gènes E2, E4, L1-L5 sont non fonctionnels. D'autres régions peuvent également être modifiées, et notamment la région E3 (WO95/02697), E2 (WO94/28938), E4 (WO94/28152, WO94/12649, WO95/02697) et L5 (WO95/02697).

Selon un mode préféré de mise en oeuvre, l'adénovirus selon l'invention comprend une délétion dans les régions E1 et E4 et la région d'ADN encadrée par les deux séquences permettant une recombinaison site-spécifique est insérée au niveau de la région E1 inactivée. Selon un autre mode de réalisation préféré, il comprend une délétion dans les régions E1 et E4 et la région d'ADN encadrée par les deux séquences permettant une recombinaison site-spécifique est insérée au niveau de la région E4 inactivée .Comme indiqué ci-après, selon un mode particulier de mise en oeuvre de l'invention, l'adénovirus comporte également une cassette d'expression du gène de la recombinase.

Les vecteurs revendiqués sont obtenus par recombinaison avec des plasmides tels que définis précédemment c'est à dire caractérisés par le fait qu'ils comprennent entre deux régions de recombinaison site-spécifiques, au moins une origine de réplication et un gène d'intérêt à activité conditionnelle.

En ce qui concerne plus particulièrement les définitions de l'origine de réplication, des deux séquences permettant une recombinaison site spécifiques et du gène d'intérêt, présents dans la région d'ADN intégrée dans le vecteur viral revendiqué, on se reportera aux définitions précitées.

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre les séquences d'ADN de l'invention, ou par construction d'un genmome viral chez E. coli. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %) ou des lignées capables de complémenter les fonctions E1 et E4 telles que décrites notamment dans les demandes n° WO 94/26914 et WO95/02697

Ensuite, les adénovirus qui se sont multipliés sont récupérés et purifiés selon les techniques classiques de biologie moléculaire, comme illustré dans les exemples.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs selon l'invention peuvent être préparés par co-transfection, dans un lignée cellulaire infectée par un virus auxiliaire humain (par exemple un adénovirus), d'un plasmide contenant les séquences nucléiques de l'invention bordée de deux régions répétées inversées (ITR) d'AAV, et d'un plasmide portant les gènes d'encapsidation (gènes rep et cap) d'AAV. Les AAV recombinants produits sont ensuite purifiés par des techniques classiques.

Concernant les virus de l'herpès et les rétrovirus, la construction de vecteurs recombinants a été largement décrite dans la littérature : voir notamment Breakfield et al., New Biologist 3 (1991) 203; EP 453242, EP178220, Bernstein et al. Genet. Eng. 7 (1985) 235; McCormick, BioTechnology 3 (1985) 689, etc.

En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus"; encore désigné MoMLV), le MSV ("murine moloney sarcoma virus"), le HaSV ("harvey sarcoma virus"); le SNV ("spleen necrosis virus"); le RSV ("rous sarcoma virus") ou encore le virus de Friend.

Pour construire des rétrovirus recombinants selon l'invention, un plasmide comportant notamment les LTR, la séquence d'encapsidation et les séquences de l'invention est généralement construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US4,861,719); la lignée PsiCRIP (WO90/02806) et la lignée GP+envAm-12 (WO89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques.

A titre de vecteurs préférés selon l'invention, on peut plus particulièrement proposer des adénovirus comprenant dans leur génome une région d'ADN conforme à l'invention et bordée par des séquences répétées inversées du bactériophage P1 (région loxP) positionnées en orientation directe.

A titre illustratif de ce type de vecteurs, on peut plus particulièrement citer les constructions suivantes avec le gène de la bgalactosidase (LacZ) ou le gène de la thymidine kinase du virus de l'Herpes (TK), (figure 1). Le gene d'interet peut etre tout gene (ADNc, ADNg, ARN, acide nucleique synthetique ou semi-synthetique) codant pour un ARN ou une proteine therapeutique ou vaccinale telle que des enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, etc; les apolipoprotéines : ApoAI, ApoAIV, ApoE, etc (FR 93 05125), la dystrophine ou une minidystrophine (FR 9111947), les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, etc, ou encore tout ou partie d'une immunoglobuline naturelle ou artificielle (Fab, ScFv, etc), un ARN ligand (WO91/19813) etc.

Le gène d'intérêt peut également être une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Les vecteurs de l'invention sont particulièrement adaptés à l'expression de séquences codant pour des facteurs toxiques. Il peut s'agir en particulier de poisons pour les cellules (toxine diphtérique, toxine pseudomonas, ricine A, etc) de produit induisant une sensibilité à un agent externe (gènes suicides :Thymidine kinase, cytosine désaminase, etc) ou encore de gènes tueurs capables d'induire la mort cellulaire (Grb3-3 (PCT/FR94/00542), ScFv anti-ras (WO94/29446), etc). Le système de l'invention permet en effet de produire des vecteurs notamment viraux contenant ces séquences sans toxicité pour les cellules de production, puis ensuite d'induire l'expression de ces molécules toxiques sélectivement dans des cellules cibles apres recombinaison site-spécifique. Ce type de construction est donc particulièrement adapté à des stratégies de thérapies antitumorales par exemple, dans lesquelles l'objectif est de détruire sélectivement les cellules affectées. Ce système est également particulièrement intéressant pour l'expression de cytokines, interférons, TNF ou TGF par exemple, dont une production incontrôlée peut avoir des effets secondaires très marqués.

La présente invention vise également toute cellule eucaryote transfectée par au moins un vecteur viral ou une molécule d'ADN, selon l'invention telle que définie ci-avant.

Un autre objet de la présente invention réside dans un procédé de production d'une molécule d'ADN telle que définie ci-avant selon lequel une culture de cellules hôtes contenant un vecteur viral selon l'invention est mise en contact avec la recombinase permettant d'induire la recombinaison site-spécifique.

Plus précisément la présente invention concerne de manière générale tout procédé de préparation caractérisé en ce qu'il met en présence:
(i) des cellules hôtes modifiées contenant au moins un vecteur viral, ledit vecteur comprenant dans son génome au moins une région d'ADN, encadrée par deux séquences permettant une recombinaison site-spécifique et positionnées en orientation directe, ladite région comprenant au moins une origine de réplication et un gène d'intérêt et
(ii) la recombinase permettant d'induire in situ la recombinaison site-spécifique, en vue de générer lesdites molécules d'ADN circulaires et réplicatives.

Différents protocoles peuvent être proposés dans le cadre de la présente invention pour effectuer cette mise en contact du vecteur viral avec la recombinase spécifique. Elle peut notamment être réalisée au niveau de la cellule hôte soit par co-transfection avec un plasmide ou co-infection avec un vecteur viral contenant le gène de la dite recombinase; soit par induction de l'expression d'un gène codant pour la dite recombinase directement présent dans le génome de ladite cellule hôte. Le gène codant pour la recombinase peut donc être présent dans la cellule hôte sous une forme intégrée au génome, sur un plasmide ou encore sur un vecteur viral annexe de type adénovirus par exemple. Dans ce cas, le vecteur viral mis en oeuvre en vu de générer la molécule d'ADN selon l'invention, est tel que défini ci-avant.

Selon une autre méthode, la cassette d'expression du gène est portée sur le vecteur viral également responsable de l'expression du gène d'intérêt.

Dans ce cas particulier, le procédé selon l'invention met en oeuvre un vecteur viral comprenant dans son génome outre une région d'ADN délimitée par deux séquences codant pour des sites de recombinaison spécifique et comprenant au moins une origine de réplication et un gène d'intérêt, une cassette d'expression du gène de la recombinase.

Un tel vecteur constitue un autre objet de la présente invention.

A cet égard, selon une variante particulière, l'invention concerne un adénovirus comportant une première délétion dans la région E1 dans laquelle est insérée le réplicon (la région d'ADN encadrée par deux séquences de recombinaison site-spécifique) et une délétion réalisée en E4 et/ou en E3 au niveau de laquelle est insérée la cassette d'expression de la protéine recombinase.

De manière réciproque au mode de mise en oeuvre précédemment décrit, le réplicon peut être inséré dans la partie délétée correspondant à la région E3 ou E4 tandis que la cassette d'expression de la recombinase est insérée au niveau de la région E1 délétée.

Selon une autre variante, le réplicon ainsi que la cassette d'expression de la recombinase sont insérés au niveau de la région E1 défective.

Plus particulièrement, comme indiqué ci-avant, les séquences permettant la recombinaison site-spécifique sont les séquences LoxP et la recombinase est la protéine Cre, dont le mode d'intetvention sur lesdites séquences de recombinaison a été décrit plus haut.

Selon un mode préféré de l'invention, il serait par ailleurs souhaitable de pouvoir contrôler et en particulier d'induire l'expression de cette recombinase au sein de la cellule hôte. A ces fins, il est avantageusement proposé dans le cadre de la présente invention de contrôler l'expression du gène codant pour la recombinase. Pour ce faire il est proposé de placer l'expression dudit gène sous contrôle d'un élément régulateur. Il peut notamment s'agir d'un promoteur inductible, permettant de contrôler les niveaux et/ou les périodes d'expression de ce gène comme par exemple le promoteur du LTR de MMTV (Pharmacia), qui est induit par la déxaméthasone ou un promoteur régulé par la tétracycline (WO94/29442; WO94/04672). Il est entendu que d'autres promoteurs peuvent être utilisés, et notamment des variants du LTR de MMTV portant par exemple des régions hétérologues de régulation (régions "enhancer" notamment).

Dans un autre mode de réalisation, l'expression du gène codant pour la recombinase est sous le contrôle de promoteurs régulés afin d'éviter soit une accumulation constitutive de ladite protéine dans la cellule hôte, ou pour minimiser la "fuite" vers le compartiment nucléaire et une certaine cylotoxicité. Il peut ainsi leur être associés des éléments qui fonctionnent comme des domaines de transactivation de transcription. A titre représentatif de ce type d'éléments on peut notamment citer les récepteurs d'hormones incluant les récepteurs stéroïdes, d'acide rétinoïque et thyroïdes parmi lesquels on peut plus particulièrement citer ceux des glucocorticoïdes, minéralcorticoïdes, thyroïdes, oestrogène, aldostérone et acide rétinoïque. Ce type de construction entre la recombinase Cre et le domaine de liaison à l'ADN d'un récepteur au glucocorticoïde a été décrit par exemple dans Feil et al. (PNAS 93 (1996) 10887).

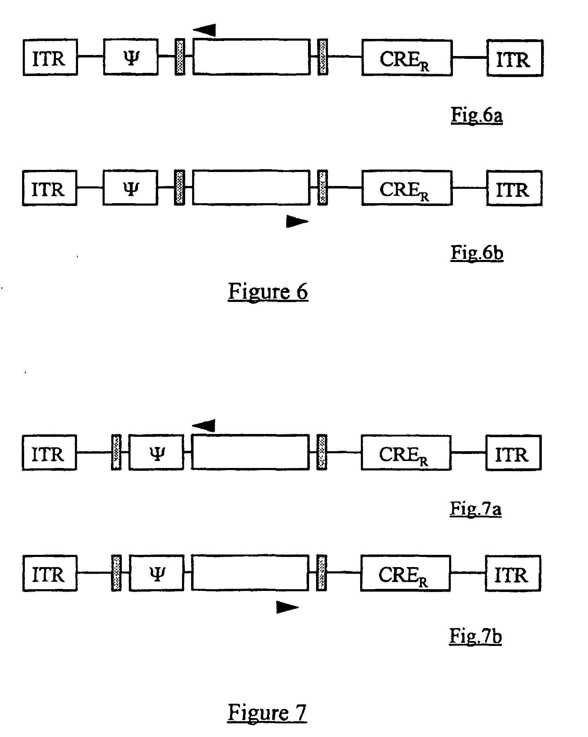

La possibilité de réguler l'expresion de la recombinase est particulièrement importante dans le cas des vecteurs de l'invention portant à la fois le réplicon et la cassette d'expression de la recombinase. En effet, dans ce mode de réalisation, si la recombinase est exprimée par exemple lors de la production des vecteurs viraux, le réplicon va être excisé du génome viral avant son encapsidation. Pour cette raison, il est utile de pouvoir disposer d'un système dans lequel l'expression de la protéine recombinase est réprimée dans les cellules de production de virus. Ceci est obtenu comme indiqué ci-avant en utilisant un promoteur régulé (type tétracycline ou MMTV), et/ou un élément de type récepteur d'hormone qui, associé à la recombinase, maintient celle-ci dans les compartiments extranucléaires en l'absence de ladite hormone (Figures 6 et 7). De ce fait, en l'absence de l'hormone, la recombinase ne peut agir, et, en présence de l'hormone, celle-ci est alors transportée vers le compartiment nucléaire où elle exerce son activité.

Une approche intéressante pour contrôler l'expression de la recombinase consiste à utilise une recombinase fusionnée à un récepteur hormonal (domaine de liaison à l'ADN), et donc inactive en l'absence de ladite hormone, puis à placer ledit gène dans le vecteur viral de telle sorte qu'il soit sous le contrôle du promoteur du réplicon. Ce mode de réalisation est représenté par exemple sur la figure 6b.

Par ailleurs, pour augmenter la sécurité du système, il est également possible, comme indiqué ci-avant, d'inclure dans le réplicon la région d'encapsidation du vecteur viral (figure 7). Ceci permet d'éviter que le stock de virus produit soit contaminé par des vecteurs ayant perdu le réplicon. En effet, si en dépit des systèmes de régulation évoqués ci-dessus, une expression de la recombinase active intervient au cours de la production du virus, cela va entraîner l'excision du réplicon à partir du génome viral, et ainsi la génération de génomes viraux dépourvus de réplicon. Si la région d'encapsidation du virus est portée par ledit réplicon, les génomes viraux ainsi générés ne seront pas encapsidés. Ainsi, seuls les génomes viraux portant à la fois le réplicon et la cassette d'expression de la recombinase peuvent être encapsidés.

La présente invention a également pour objet des compositions pharmaceutiques comprenant au moins un vecteur viral selon l'invention ou une cellule transfectée selon l'invention. Ces compositions peuvent être formulées en vue d'une administration par voie topique, orale, parentérale, intranasale, intraveineuse, intramusculaire, sous-cutanée, intraoculaire, etc. Préférentiellement, la composition selon l'invention contient des véhicules pharmaceutiquement acceptables pour une formulation injectable. II peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables. S'agissant des rétrovirus, ils peut être avantageux d'utiliser directement les cellules d'encapsidation ou des cellules infectées ex vivo en vue de leur réimplantation in vivo, éventuellement sous forme de néo-organes (WO94/24298).

Les doses de vecteur utilisées pour l'injection peuvent être adaptées en fonction de différents paramètres, et notamment en fonction du mode d'administration utilisé, de la pathologie concernée ou encore de la durée du traitement recherchée. D'une manière générale, les virus recombinants selon l'invention sont formulés et administrés sous forme de doses comprises entre 10⁴ et 10¹⁴ pfu/ml. Pour les AAV et les adénovirus, des doses de 10⁶ à 10¹⁰ pfu/ml peuvent également être utilisées. Le terme pfu ("plaque forming unit") correspond au pouvoir infectieux d'une suspension de virions, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

Selon le gène d'intérêt, présent dans les molécules d'ADN de l'invention ou les régions intégrées dans le gènome desdits vecteurs viraux ceux-ci peuvent être utilisées pour le traitement ou la prévention de nombreuses pathologies, incluant les maladies génétiques (myodystrophie, mucoviscidose, etc), les maladies neurodégénératives (Alzheimer, Parkinson, ALS, etc), les cancers, les pathologies liées aux désordres de la coagulation ou aux dyslipoprotéinémies, les pathologies liées aux infections virales (hépatites, SIDA, etc), ou dans les domaines agronomique et vétérinaire, etc.

La présente invention sera plus complètement décrite à l'aide des exemples qui suivent, qui doivent être considérés comme illustratifs et non limitatifs.

### Légende des figures

Tableau 1: Origine des séquences utilisées pour la construction de l'épisome
Figure 1: Schéma de construction de l'épisome
Figure 2: Representation de la structure de l'épisome.
Figure 3: Séquence entre le promoteur (P-RSV) et la cassette d'expression (TK-CITE-EBNA1) dans le plasmide pLoxP-ori-TK-EBNA1 (A) et dans l'épisome après recombinaison (B, C et D) - SEQ ID n° 3
Figurer 4: Schéma des étapes de clonage de la cassette LoxP-ori-TK-EBNA1
Figure 5: Schéma des étapes de clonage de la cassette LoxP-ori-LacZ-EBNA1
Figure 6 : Représentation d'un vecteur adénoviral portant un réplicon et une cassette d'expression de Cre régulée : CRE_{R} : Cre régulée, soit au niveau du promoteur, soit par une fusion, soit les deux. En (b), l'orientation du réplicon permet de placer la cassette CRE_{R} sous contrôle du promoteur présent dans le réplicon. La boite grise correspond aux sites LoxP.
Figure 7 : Représentation d'un vecteur adénoviral portant un réplicon et une cassette d'expression de Cre régulée, la région d'encapsidation virale Psi (Ψ) étant incluse dans le réplicon. La boite grise correspond aux sites LoxP.

### Techniques générales de clonage et de biologie moléculaire

Les méthodes classiques de biologie moléculaire telles que la centrifugation d'ADN plasmidique en gradient de chlorure de césium-bromure d'éthidium, les digestions par des enzymes de restriction, l'électrophorèse sur gel, l'électroélution des fragments d'ADN à partir de gels d'agarose, la transformation dans E. coli, la précipitation des acides nucléiques etc, sont décrites dans la littérature (Maniatis et al., 1989, Ausubel et al., 1987). Les séquences nucléotidiques ont été déterminées par la méthode de terminaison de chaînes en suivant le protocole déjà présenté (Ausubel et al., 1987).

Les enzymes de restriction ont été fournies par New-England Biolabs (Biolabs), Bethesda Research Laboratories (BRL) ou Amersham Ltd (Amersham).

Pour les ligatures, les fragments d'ADN sont séparés selon leur taille sur des gels d'agarose à 0,7 % ou d'acrylamide à 8 %, purifiés par électrophorèse puis électroélution, extraits au phénol, précipités à l'éthanol puis incubés dans un tampon Tris-HCl pH 7.4 50 mM, MgCl₂ 10 mM, DTT 10 mM, ATP 2 mM, en présence d'ADN ligase du phage T4 (Biolabs). Les oligonucléotides sont synthétisés en utilisant la chimie des phosphoramidites protégés en b par un groupement cyanoéthyl (Sinha et al., 1984, Giles 1985) avec le synthétiseur automatique d'ADN Biosearch 8600 en utilisant les recommandations du fabricant.

Les ADN plasmidiques sont purifiés suivant la technique de lyse alcaline (Maniatis et al., 1989).

### EXEMPLES

### Exemple 1. Description d'un vecteur conforme à l'invention (figures 1-3)

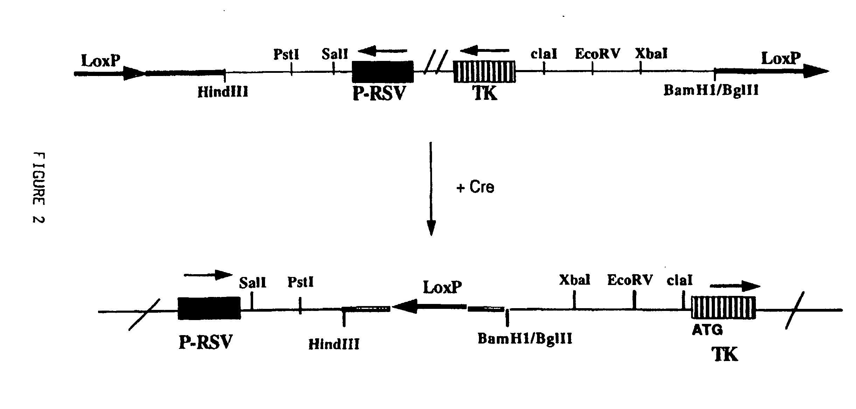

La Figure 1 decrit la structure generale d'un vecteur selon l'invention et, plus particulierement, de la region comprise entre les sequences permettant la recombinaison site-specifique. Une construction plus detaillee est donnee sur la figure 2.

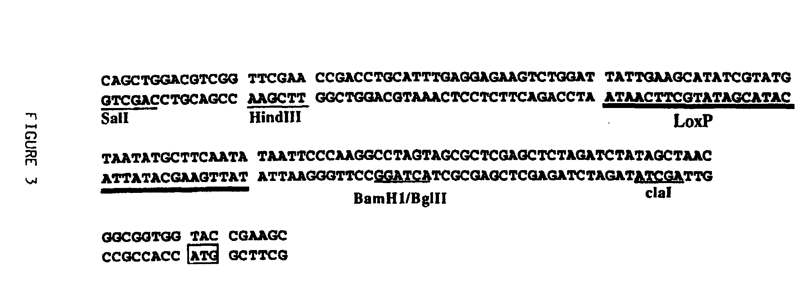

L'orientation du promoteur (P) et de la cassette d'expression du transgène (TG) et de la protéine EBNA1 est indiquée par des flèches et ne permet donc l'expression de ces deux gènes qu'après recombinaison en présence de la recombinase Cre. Le détail des séquences entre le promoteur (P) et le gene apres recombinaison est présenté dans la figure 3. Sur cette figure, le promoteur est le LTR du virus RSV (P-RSV) et le gène est le gene de la thymidine kinase TK. Le premier ATG de l'ARN messager, correspondant à celui du gène TK, est souligné. Comme illustre sur la figure 1, l'expression d'EBNA-1 est obtenue à partir d'un messager polycistronique par initiation interne de la traduction en utilisant la séquence IRES (Internal Ribosome Entry Site) aussi appelée séquence CITE ("Cap Independent Translation Entry") du virus de l'Encephalomyocardite (ECMV). La séquence signal de fin de transcription et de polyadénylation du virus SV40 (pA) a été introduite à l'extrémité 3' de la séquence codante d'EBNA1. La séquence oriP est placée entre la cassette d'expression TK-EBNA-1 et le promoteur RSV. Cette séquence n'est active pour la réplication qu'en présence d'EBNA-1, par conséquent, elle ne fonctionne qu'après recombinaison et formation de l'épisome.

### Exemple 2. Construction des plasmides LoxP-oriP-TK-EBNA1 et LoxP-ori-LacZ-EBNA1

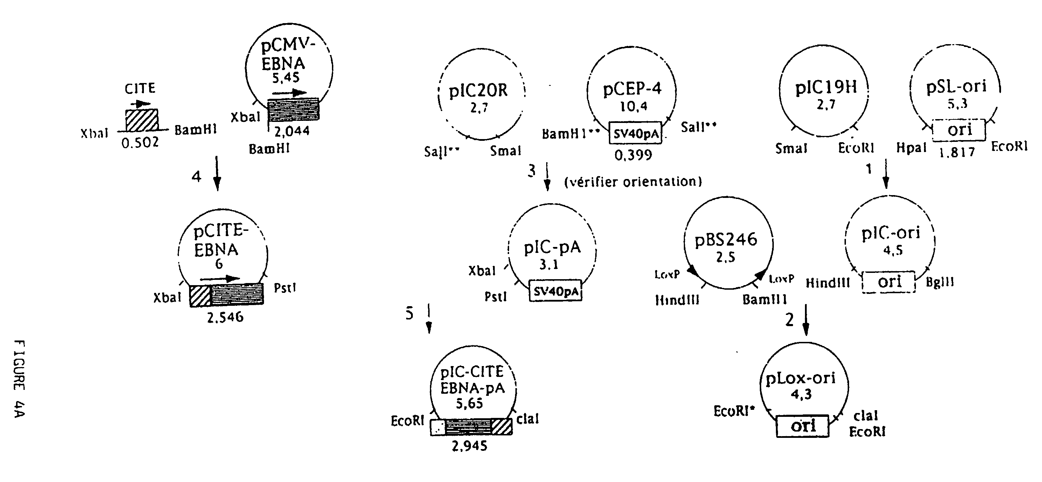

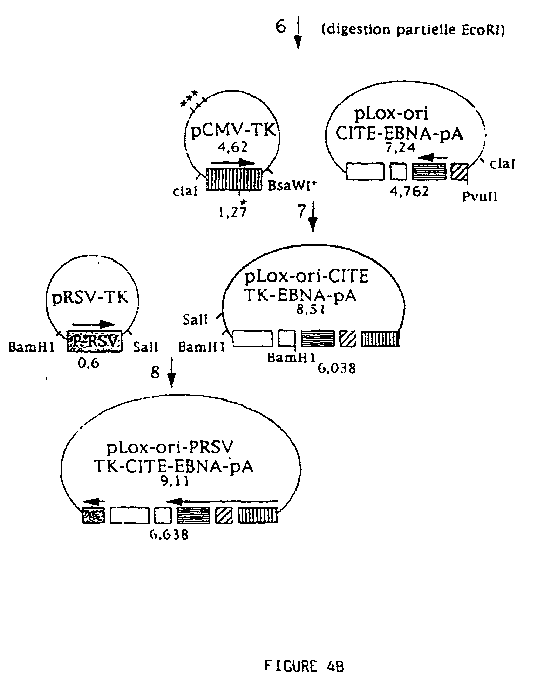

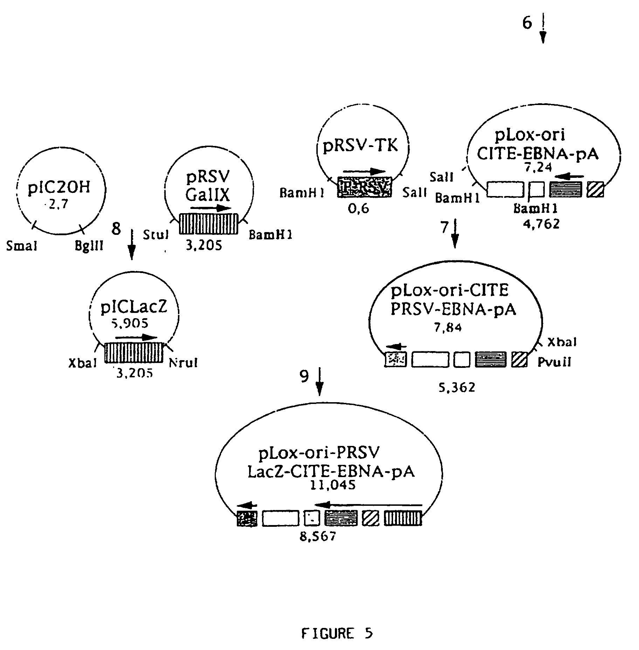

Les étapes de la construction des plasmide sont présentées dans les figures 4 et 5 et l'origine des séquences utilisées est résumée dans le tableau 1.

### 2.1. Construction du plasmide pLoxP-oriP-TK-EBNA1 (Figure4)

1. Le plasmide pSLori a ete digere avec les enzymes de restriction HpaI et EcoRI et le fragment de 1817 bp obtenu, correspondant a la sequence d'oriP, a ete clone entre les sites SmaI et EcoRI du plasmide pIC19H prealablement dephosphoryle, pour donner le plasmide pIC-ori (Figure 4A).
2. Le plasmide pIC-ori a ete digere aux sites HindIII et BglII, dephosphoryle, et le fragment de 1900 pb obtenu a ete clone entre les sites HindIII (174) et BamHI (208) du vecteur pBS246 pour donner le vecteur pLox-ori (Figure 4A).
3. Le fragment BamHI-SalI de 399 bp du vecteur pCEP4, correspondant a la sequence du signal d'arret de transcription et de polyadenylation du virus SV40, a ete repare avec l'ADN polymerase Klenow puis clone entre les sites SmaI et SalI (repare avec l'ADN polymerase Klenow) du vecteur pIC20R prealablement dephosphoryle, pour donner le vecteur pICpA (Figure 4A).
4. La sequence IRES d'ECMV comprise entre les nucleotides 16 et 518 du vecteur pCITE-2 a ete amplifiee par PCR à l'aide des oligonucléotides synthétiques 1 5'-GGCCTCTAGACAGCTGGTTATTTTCC-3' (SEQ ID n° 1) et 2 5'-GGCCGGATCCCATATTATCATCG-3' (SEQ ID n° 2) comprenant les sites XbaI et PvuII (oligo 1) et BamHI (oligo 2) a leur extremite 5'. Le produit obtenu a ete digere avec les enzymes de restriction XbaI et BamHI et clone entre les sites XbaI et PstI du vecteur pCMV-EBNA1 préalablement dephosphoryle, pour donner le vecteur pCITE-EBNA1. La sequence comprise entre l'ATG 12 de l'IRES, correspondant au codon d'initiation de la traduction, et le codon serine suivant le codon methionine de la proteine EBNA1, a ete deletee par mutagenese dirigee a l'aide de la technique PCR (ex-site PCR). La sequence complete de l'IRES et d'EBNA1 obtenue apres PCR a ete entierement sequencee (Figure 4A).
5. Le fragment XbaI-PstI du vecteur pCITE-EBNA1 (2546 bp) a ete clone entre les sites Xbal et PstI du plasmide pICpA prealablement dephosphoryle, pour donner le vecteur pCITE-EBNA1-pA (Figure 4A).
6. Le fragment ClaI-EcoRI du vecteur pCITE-EBNA1-pA (2945 pb) a ete clone dans le vecteur pLox-ori, digere de facon partielle au site EcoRI situe a l'extremite d'ori, puis digere avec ClaI et dephosphoryle pour donner le vecteur pLox-ori-CITE-EBNA1-pA (Figure 4B).
7. Le fragment ClaI-BsaW1 (1270 pb) du vecteur pCMV-TK, obtenu par digestion partielle avec BsaW1 et reparation du site BsaW1 avec l'ADN polymerase Klenow, a ete clone entre les sites ClaI et PvuII du vecteur pLox-ori-CITE-EBNA1-pA pour donner le vecteur pLox-ori-CITE-TK-EBNA1-pA (Figure 4B).
8. Le fragment BarnHI-SalI (600 pb) du plasmide pRSV-TK, correspondant a la sequence du promoteur du LTR de RSV, a ete clone entre les sites BamHI et SalI du vecteur pLox-ori-CITE-TK-EBNA1-pA, prealablement dephosphoryle, pour donner le plasmide pLox-ori-pRSV-TK-CITE-EBNA1-pA (Figure 4B).

### 2.2. Construction du plasmide pLoxP-oriP-LacZ-EBNA1 (Figure 5)

Les clonages 1-6 decrits dans l'exemple 2.1 ci-dessus et illustres sur la figure 4A sont communs a la construction des vecteurs pLox-ori-pRSV-TK-CITE-EBNA1-pA et pLox-ori-pRSV-LacZ-CITE-EBNA1-pA.
7. Le clonage du promoteur du LTR de RSV a ete effectue selon l'étape 7. de l'exemple 2.1. (Figure 5).
8. La fragment BamHI-StuI (3205 pb) du vecteur pRSV-GalIX, correspondant au gene LacZ precede d'un signal de localisation nucleaire (NLS), a ete clone entre les sites SmaI et BglII du vecteur pIC20H, prealablement dephosphoryle, pour donner le plasmide pICLacZ (Figure 5).
9. Le fragment XbaI-NruI (3205 pb) du vecteur pICLacZ a ete clone entre les sites XbaI et PvuII du vecteur pLox-ori-pRSV-CITE-EBNA1-pA pour donner le vecteur pLox-ori-pRSV-LacZ-CITE-EBNA1-pA (Figure 5).

### Exemple 3. Validation du système par co-transfection de cellules humaines (Hela-EBNA1; 143B-TK-) à l'aide des plasmides CMV-Cre (pBS185) et LoxP-oriP-TK-EBNA. 1 ou LoxP-oriP-LacZ-EBNA1.

### 3.1. Validation in vitro

Une lignée de cellules Hela exprimant le gène EBNA1 de façon stable a ete transfectée avec les plasmides pLoxP-oriP-LacZ-EBNA1 et un plasmide exprimant le gene de la recombinase Cre sous le controle du promoteur du cytomegalovirus (pBS185). L'efficacité de recombinaison et la fonctionnalité de la cassette d'expression du gène LacZ ont ete évaluées par l'activité bgalactosidase dans les cellules, observee uniquement en presence de la recombinase Cre. Les resultats obtenus démontrent l'efficacité de génération du réplicon sous l'action de la recombinase Cre, mise en évidence par l'activation de l'expression du gène LacZ après recombinaison entre les sites LoxP et formation de l'épisome. De plus, apres 3 semaines de culture des cellules co-transfectees [passage une fois par semaine (dilution 1:10)], on observe un maintien de la proportion de cellules exprimant le gene LacZ, alors que l'expression de LacZ a disparu dans les cellules transfectees avec le plasmide controle ne possedant pas l'origine de replication oriP, ce qui demontre le caractere fonctionnel d'oriP dans la construction.

De la meme facon, une lignée de cellules TK⁻ (143TK⁻) est co-transfectée par les plasmides LoxP-ori-TK-EBNA1 et pBS185. Les cellules transfectées exprimant le gène TK sont sélectionnées en milieu HAT. La stabilité de l'expression du gène TK au cours des divisions cellulaires et donc l'activité du système oriP-EBNA1 est vérifiée par immunofluorescence, à l'aide d'un anticorps monoclonal spécifique de la protéine TK du virus de l'Herpès. La présence de l'épisome et sa réplication au cours des divisions cellulaires est mise en évidence par la technique du Hirt, suivie d'une amplification de l'ADN épisornique par la technique PCR, à l'aide d'amorces spécifiques.

### 3.2. Validation in vivo

L'activite de ces constructions in vivo est testee par injection intratumorale (electroporation) d'ADN des plasmides pLoxP-ori-TK-EBNA1 ou pLoxP-ori-LacZ-EBNA1 et pBS185 dans des tumeurs induites par injection sous-cutannee de cellules Hela ou Hela-EBNA1 chez la souris nude. L'injection de cellules prealablement co-transfectees avec ces memes plasmides est effectuee en parallele. Le maintien du transgene (TK ou LacZ) dans les tumeurs transduites par ces constructions est analyse par imrnunohistochimie, par comparaison a un plasmide controle ne possedant pas l'origine de replication.

### Exemple 4. Analyse du système de recombinaison et de maintien épisomal.

### 4.1- Construction du plasmide pCre

Le plasmide p-Cre contient le gène de la recombinase Cre fusionné en 5' au signal de localisation nucléaire de l'antigène T du virus SV40 et exprimé à partir du promoteur de la thymidine kinase (TK) du virus de l'herpès. Une construction similaire est réalisée portant, à la place du promoteur TK, un promoteur régulé par la tétracycline ou le promoteur MMTV. Par ailleurs, une cassette portant une fusion Cre-ER est également réalisée.

### 4.2- Construction du plasmide pRep

Le plasmide réplicon (pRep) contenant une fusion du gène de résistance à la phléomycine avec le gène de la βgalactosidase (Zéo-LacZ), oriP et le promoteur majeur précoce du cytomégalovirus (P.CMV), sans la protéine EBNA1 a été construit comme suit.

Le fragment StuI-claI (1149-4553) du plasmide pRSV-Gal-IX contenant le gène LacZ et le signal de polyadénylation du virus SV40 a été cloné entre les sites Clal et EcoRV (2029-2032) de pLox-ori (exemple 2 et figure 4) pour donner le plasmide pLox-ori-LacZ.
Le fragment BgIII-BgIII (473-1226) de pCEP4 contenant le promoteur précoce de CMV a été cloné au site BamH1 de pLox-ori-LacZ pour donner pLX2.
L'extrémité 5' du gène LacZ dans pLX2 (fragment Xba1-ClaI) a été substituée par le fragment NcoI-claI du plasmide pUT651 (CAYLA, tableau 1). Pour faciliter le clonage ce fragment a préalablement été sous-cloné entre les sites EcoRV et claI du plasmide pIC20RpA puis digéré par XbaI et claI et cloné entre les sites XbaI et ClaI de pLX2.
Le plasmide contrôle contient la même stucture que pRep dépourvue de l'origine de réplication (OriP).

### 4.3- Construction du plasmide pRep-EBNA1

La cassette permettant l'expression d'EBNA1 à partir de l'IRES d'ECMV a été construite par mutagénèse dirigée à partir du plasmide pCITE-EBNA1-pA et clonée dans le vecteur navette de l'Adénovirus pAdLX2 (5-2-2) pour donner pAdLX2-EBNA1 ou pRep-EBNA1. L'IRES ainsi que le début d'EBNA1 ont été entièrement séquencés. L'expression d'EBNA1 à partir de l'IRES a été analysée par Western-Blot dans les cellules Hela transfectées par pRep-EBNA1. Le plasmide pCMV-EBNA1 qui a servi à la construction de pCITE-EBNA1pA sert de controle.

### 4.4- Validation in vitro

### 4.4.1-Analyse de la recombinaison et du maintien épisomal dans les cellules Hela-EBNA1

Dans une première étape, des expériences de transfection de cellules Hela exprimant la protéine EBNA1 de façon constitutive (Hela-EBNA1) avec pRep seul ou avec pCre ont montré que la co-transfection des deux plasmides permettait l'excision du réplicon et l'induction de l'expression du gène de la βgalactosidase. Les cellules controle transfectées avec le plasmide réplicon seul, montrent un bruit de fond constant mais quasi négligeable d'activité β galactosidase. L'expression du transgène a été suivie au cours des passages successifs des cellules, à raison de 2 repiquages par semaine pendant un mois, ce qui équivaut à 24 divisions cellulaires. Dans les cellules transfectées avec pRep+pCre, l'expression de la βgalactosidase s'est maintenue pendant toute la durée de l'expérience alors que celle-ci a été rapidement perdue (après quelques divisions cellulaires) dans les cellules cotransfectées avec le plasmide controle ne portant pas l'origine de réplication oriP.

Dans une seconde étape, les cellules co-transfectées par (pRep+ pCre) ont été sélectionnées en présence de phléomycine. L'ADN épisomal a été isolé par la technique de Hirt, linéarisé ou non par digestion au niveau du site unique XhoI puis éventuellement digéré par MboI pour démontrer que l'ADN a bien répliqué dans les cellules eucaryotes. L'analyse par Southern-Blot des échantillons ainsi obtenus a permis de mettre en évidence, la présence d'un réplicon de la taille attendue. L'estimation du nombre de copies par cellules est de 1 à 10. Les cellules sélectionnées ont ensuite été maintenues en présence ou en absence de pression de sélection. Dans ces deux conditions, on observe un maintien similaire de l'expression de la βgalactosidase avec une décroissance lente et constante au cours des divisions cellulaires. Après 27 divisions, une activité βgalactosidase est encore détectable dans 25% des cellules par coloration directe in vitro. Ces résultats correspondent à une stabilité ségrégationnelle de l'épisome de 97% par division. Ils sont en accord avec les résultats publiés, en effet un taux de perte de 1 à 5 % par division a été rapporté (Simpson et coll., 1996).

### 4.4.2- Analyse de la fonctionalité d'EBNA1 dans les cellules Hela

Des expériences de co-transfection de cellules Hela avec pRep-EBNA1 et pCre permettent de tester qu'EBNA1 est correctement exprimée à partir de l'IRES et assure le maintien de l'expression de la βgalactosidase au cours des passages successifs. Les cellules Hela-EBNA1 transfectées avec les mêmes plasmides servant de controle.

### 4.5. Validation in vivo dans un modèle de tumeur induite chez la souris nude.

Les cellules Hela et Hela-EBNA1 sont tumorigènes chez la souris nude, l'injection sous-cutanée de 10⁶ cellules induit la formation d'une tumeur détectable en 8 jours qui croît très rapidement, et peut-être suivie pendant un mois. Dans une première expérience les cellules Hela EBNA1 transfectées par pRep et pCre puis sélectionnées in vitro en présence de phléomycine ont été injectées, L'analyse des tumeurs (3cm de diamètre) à 21 jours, après coloration au X-gal démontre le maintien du réplicon in vivo dans ce modèle.

Dans une seconde expérience, les cellules Hela-EBNA1 transfectées par pRep puis infectées par l'AdCre (cf exemple 5.2) ont été injectées, Des cellules transfectées avec deux plasmides dérivés de pRep permettant l'expression de lacZ en absence de recombinaison et portant ou non oriP, injectées simultanément, servent de contrôle. L'analyse des tumeurs à 21 jours démontre que l'adénovirus Cre de troisième génération permet l'excison du réplicon et que sa présence n'altère ni la viabilité cellulaire ni les premières étapes de l'établissement de l'épisome. A noter qu'aucune activité β galactosidase n'était détectable dans les cellules transfectées avec le plasmide contrôle ne portant pas oriP. Ces résultats montrent clairement que les constructions selon l'invention sont fonctionnelles in vivo dans des cellules tumorales, on note également une stabilité des épisomes après 21 jours.

### Exemple 5. Construction des adénovirus recombinés de 1ère génération et de 3ième génération.

Cet exemple décrit la construction de vecteurs viraux selon l'invention, comprenant une région pouvant générer, par recombinaison site-specifique, une molécule circulaire et réplicative in vivo. Ces vecteurs viraux comprennent en outre une séquence codant pour la recombinase permettant la recombinaison.

### 5.1. Préparation d'adenovirus de lère génération (à partir des plasmides pBS185, pLoxP-oriP-TK-EBNA1 et pLoxP-oriP-LacZ-EBNA1).

La cassette d'expression de la recombinase Cre et la séquence complète du réplicon incluant les sites LoxP sont clonés à partir des vecteurs pBS185 et pLox-oriP-TK-EBNA1 ou pLoxP-oriP-LacZ-EBNA1 dans la région E1 de vecteurs adénoviraux comprenant une déletion de tout ou partie de la région E1 (Add1327; ΔE1-ΔE3). Les adénovirus recombinants sont isolés par les techniques classiques de recombinaison homologue dans les cellules 293. Les vecteurs viraux peuvent également être préparés par double recombinaison dans E.Coli à l'aide de plasmides contenant le génome de l'Adéno 5 ΔE1ΔE3, le génome viral étant ensuite encapsule dans une particule adénovirale dans une lignée appropriée. Pour des raisons de capacité de clonage, le gène LacZ est avantageusement remplacé par un gène marqueur plus petit.

### 5.2. Préparation d'adenovirus de 3ème génération

L'utilisation d'adénovirus de 3ème génération présente certains avantages par rapport à ceux de lère génération; non seulement sur le plan de l'innocuité mais aussi de la diminution de la réponse inflammatoire et de l'augmentation de la stabilité de l'expression du transgène. Ces vecteurs présentent en outre une capacité de clonage augmentée et une absence d'effet cytopathique direct in vitro et in vivo.

Les vecteurs de 3ième génération (Ad11007; ΔE1ΔE3ΔE4) peuvent également être prépares par les techniques classiques de recombinaison homologue dans les cellules d'empaquetage appropriées (WO 96/22378) ou par double recombinaison dans E.Coli puis empaquetage.

Les deux adénovirus ont été construits par double recombinaison à l'aide des squelettes E. Coli contenant un génome d'adénovirus de 3ème génération pXL2811 (pRSV-bGal-ΔE1,ΔE3,ΔE4-dl1007-SspI) et pXL2789 (pΔE1,ΔE3,ΔE4-dl1007-SspI), et des plasmides suicide (Kana-SacB) pMA37 ou pXL3048, destinés à modifier la région E1 selon la stratégie décrite précédemment (Crouzet et coll., 1997 PNAS, 94:1414; WO96/25506).

### 5.2.1. Préparation d'adenovirus Cre (Ad-Cre)

Le fragment XhoI-BamHI (451-2057) du plasmide pMC-Cre (tableau 1) a été réparé avec la Klenow et cloné au site EcoRV du plasmide suicide pMA37. Le génome de l'adénovirus obtenu par double recombinaison avec le plasmide pXL2811 a été linéarisé par digestion avec PacI et transfecté dans les cellules IGRP2 (WO96/22378) à l'aide de Lipofectamine (GIBCO). L'Ad-Cre 3.0 ainsi produit a été amplifié dans les mêmes cellules puis purifié selon les techniques classiques (chlorure de césium) ou par chromatographie (FR96/08164).
La structure du génome de l'adénovirus Cre a été confirmée par digestion enzymatique.

### 5.2.2. Préparation d'adenovirus Rep (Ad-Rep)

Le plasmide pLX2 a été digéré par BamH1 puis recircularisé pour éliminer le signal de polyadénylation de SV40, en 5' du gène LacZ. Le fragment NotI-NotI (1-6145) du plasmide ainsi obtenu a été réparé par Klenow puis cloné au site EcoRV du vecteur suicide pXL3048 préalablement digéré par BamH1 et SalI, réparé par Klenow puis recircularisé pour détruire ces deux sites, pour donner le plasmide pAdLX2. Le fragment XhoI-SalI (441-3457) du plasmide pCITE-EBNAlpA-mutagénisé a été cloné au site Xhol du plasmide pAdLX2 précédemment obtenu pour donner le plasmide pAdLX2-EBNA1 (pRep-EBNA1).
L'Ad-Rep a été obtenu par recombinaison avec les plasmides pAdLX2-EBNA1 et pXL2789, selon les techniques décrites précédemment pour l'Ad-Cre.

### 5.2.3. Préparation d'adenovirus Rep/Cre (Figure 6)

L'adénovirus Rep-Cre portant à la fois le réplicon et le gène de la recombinase Cre est construit. Cette stratégie permet d'augmenter l'efficacité de transfert du réplicon et tout particulièrement in vivo. La cassette d'expression de Cre est insérée dans le génome de l'adénovirus dans les régions, E1, E3 ou E4. Une expression parfaitement régulée de la recombinase est recherchée pour empêcher l'excision du réplicon à partir du génome de l'adénovirus, lors de sa propagation dans les cellules IGRP2.

La régulation de l'expression de la recombinase au niveau transcriptionnel (promoteur tissu spécifique activé in vivo ou promoteur inductible) ou au niveau post-transcriptionnel (fusion de Cre avec le domaine de fixation du récepteur aux hormones stéroïdiennes Cre-ER) a été envisagée.

Pour construire ces adénovirus, le réplicon est introduit dans le plasmide pXL2789 comme décrit dans l'exemple précédent. La cassette d'expression de Cre comportant le promoteur tet ou MMTV, ou une fusion Cre-ER est ensuite introduite par double recombinaison homologue chez E.coli dans ledit plasmide pour générer les plasmides pRep-Cre1 (Rep et Cre en E1) et pRep-Cre2 (Rep en E1 et Cre en E4). Ces plasmides sont ensuite traités par PacI pour extraire le génome viral recombinant, qui est introduit dans les cellules IGRP2 pour produire les virus correspondants.

### 5.2.4 Préparation d'adenovirus Rep/Psi (figure 7)

Cette construction permet avantageusement d'éviter la contamination de l'Ad-Rep-Cre par de l'Adénovirus délété du réplicon dans le cas ou une régulation parfaite de l'activité de Cre ne pourrait être obtenue. La stratégie repose sur l'insertion du signal d'encapsidation Psi dans le réplicon. Le vecteur pXL3048 est modifié par mutagénèse dirigée au niveau de la région ITR afin de déléter le signal d'encapsidation et d'introduire un site LoxP pour donner le plasmide pXL3048-ΔPsi-LoxP. La séquence du réplicon délétée du site « LoxP gauche » est isolée par digestion enzymatique à partir des plasmides pAdLX2 ou pAdLX2-EBNA1 et clonée au site EcoRV du plasmide pXL3048-ΔPsi-LoxP.

### Exemple 6. Validation du système par co-infection avec les deux adénovirus recombinés:

La fonctionalite des vecteurs viraux de l'invention est controlee in vitro et in vivo :

### 6.1. Validation in vitro, par infection de différentes lignées de cellulaires.

L'activité recombinase de l'adénovirus Cre a été démontrée in vitro dans les cellules Hela-EBNA1 transfectées par pRep ainsi que dans une lignée de cellules embryonnaires de souris (LoxP-bgal) dans laquelle l'expression de lacZ peut-être activée par recombinaison entre deux sites loxP.

L'efficacité de l'excision du réplicon à partir du génome adénoviral a été étudiée dans les cellules IGRP2 co-infectées par l'Ad-Rep et l'Ad-Cre.
L'analyse directe de l'ADN viral isolé par la technique de Hirt et digéré par XhoI révèle une disparition totale du fragment correspondant au réplicon non-excisé du génome de l'Ad-Rep ce qui démontre l'efficacité de la recombinaison entre les deux sites LoxP. L'analyse par Southem des mêmes échantillons a permis de mettre en évidence un fragment de 9,2 kb correspondant au réplicon.
Dans les cellules Hela co-infectées par l'Ad-Rep et Ad-Cre, on observe à 48h une activité bgalactosidase dans 50% des cellules alors qu'aucune activité bgalactosidase n'est détectable dans les cellules infectées avec l'adéno-Rep seul. A 96h, le nombre de cellules exprimant lacZ augmente avec la division cellulaire. Après passage des cellules l'expression de lacZ se maintient encore pendant au moins 20 jours après la co-infection. Ces résultats démontrent que (i) le réplicon peut-être délivré efficacement dans la cellule par co-infection avec ces deux adénovirus, que (ii) la recombinaison libère le réplicon et active l'expression du transgène et que (iii) le réplicon permet d'assurer l'expression stable du transgène au cours des divisions cellulaires.

### 6.2. Validation in vivo

La validation in vivo a été démontrée par le transfert de cellules humaines normales (kératinocytes, cellules souches hématopoïétiques (CD34+), cellules souches épithéliales bronchiques, myoblastes...) ou cancéreuses (MDA, HT29,...) préalablement co-infectées par l'Ad-Rép et l'Ad-Cre, chez la souris nude.
L'expression du transgène et la stabilité de l'épisome au cours des divisions cellulaires sont vérifiées par les techniques précédemment décrites.

### LISTE DE SEQUENCES

(1) INFORMATIONS GENERALES:
   (i) DEPOSANT:
      (A) NOM: Rhone Poulenc Rorer SA
      (B) RUE: 20, avenue Raymond Aron
      (C) VILLE: ANTONY
      (E) PAYS: France
      (F) CODE POSTAL: 920165
   (ii) TITRE DE L'INVENTION: GENERATION DE MOLECULES REPLICATIVES IN VIVO.
   (iii) NOMBRE DE SEQUENCES: 3
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 26 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 1:
   GGCCTCTAGA CAGCTGGTTA TTTTCC 26
(2) INFORMATIONS POUR LA SEQ ID NO: 2:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 2:
   GGCCGGATCC CATATTATCA TCG 23
(2) INFORMATIONS POUR LA SEQ ID NO: 3:
   (i) CARACTERISTIQUES DE LA SEQUENCE:
      (A) LONGUEUR: 147 paires de bases
      (B) TYPE: nucléotide
      (C) NOMBRE DE BRINS: double
      (D) CONFIGURATION: linéaire
   (ii) TYPE DE MOLECULE: ADNc
   (xi) DESCRIPTION DE LA SEQUENCE: SEQ ID NO: 3:

**Tableau 1 :**

| Origine des séquences utilisées pour la construction de l'épisome | | | |
|---|---|---|---|
| Sequence | Fragment | Taille (pb) | origine |
| LoxP | NotI (1-296) | 34 | pBS246 (GIBCO-BRL) |
| LTR de RSV | BamHI-SalI (478-1080) | 602 | pRSV-TK (WO95/14101) |
| oriP | EcoRI-HpaI (5052-3235) | 1817 | pSLori (WO95/14101) |
| SV40 polyA | BamHI-SalI (406-7) | 399 | pCEP-4 (In Vitrogen) |
| EBNA-1 | BamHI-PstI (759-2803) | 2044 | pCMV-EBNA (Clontech) |
| IRES | (16-518) | 502 | pCITE 2a (Novagen) |
| TK | claI-BsawI (1721-556) | 1165 | pCMV-TK-E1 Gene Therapy 3 (1996) 315) |
| pIC19H | | 2700 | Marsh et al., Gene 32 (1984) 481 |
| pIC20H | | 2700 | |
| pIC20R | | 2700 | |
| pBluescriptII-KS | | 2850 | (Stratagene) |
| hCMV | BgIII-BgIII (473-1226) | 753 | pCEP4 (IN VITROGEN) |
| LacZ | StuI-BamH1 (1149-4354) | 3205 | pRSVGalIX (L.Stratford-Perricaudet, J.Clin.Invest 1992 p 626) |
| hCMV-Cre-MTpA | HindIII-HindIII (0-3400) | 3400 | pBS185 (GIBCO-BRL) |
| Zéo-Lac | NcoI-ClaI (750-1980) | 1230 | pUT641 (CAYLA) |

## Revendications

1. Vecteur viral **caractérisé en ce qu'**il comprend une région d'ADN délimitée par deux séquences permettant une recombinaison site-spécifique et positionnées en orientation directe, ladite région comprenant successivement un promoteur fonctionnel dans les cellules mammifères, la séquence oriP du virus EBV, une cassette d'expression comportant un gène d'intérêt et le gène de la protéine EBNA1 séparés par une séquence IRES dérivée du virus ECMV, le promoteur et la cassette d'expression étant orientés de telle sorte que l'expression des deux gènes n'est possible qu'après la recombinaison site-spécifique.

2. Vecteur viral selon la revendication 1 **caractérisé en ce que** l'expression du gène d'intérêt est dépendante de la recombinaison site-spécifique.

3. Vecteur viral selon l'une des revendications 1 et 2 **caractérisé en ce que** les séquences permettant la recombinaison site-spécifique sont des séquences capables de recombiner spécifiquement en présence d'une recombinase.

4. Vecteur viral selon la revendication 3 **caractérisé en ce que** les séquences permettant la recombinaison site-spécifique sont dérivées d'un bactériophage.

5. Vecteur viral selon la revendication 4 **caractérisé en ce que** les séquences permettant la recombinaison site-spécifique sont dérivées du bactériophage P1.

6. Vecteur viral selon la revendication 5 **caractérisé en ce qu'**il s'agit des séquences répétées inversées du bactériophage P1 (région loxP), dont la recombinaison est induite par la recombinase Cre.

7. Vecteur viral selon la revendication 3 **caractérisé en ce qu'**il comprend en outre, hors de la région délimitée par les deux séquences permettant une recombinaison site spécifique, le gène codant pour la recombinase correspondante.

8. Vecteur viral selon la revendication 7 **caractérisé en ce que** le gène codant pour la recombinase est placé sous contrôle d'un promoteur inductible.

9. Vecteur viral selon la revendication 8 **caractérisé en ce que** le promoteur est choisi parmi le promoteur MMTV inductible par la déxaméthasone et un promoteur inductible par la tétracycline.

10. Vecteur viral selon la revendication 8 **caractérisé en ce que** le gène de la recombinase comprend en outre un élément régulateur codant pour le domaine de liaison d'un récepteur d'une hormone.

11. Vecteur viral selon la revendication 10 **caractérisé en ce qu'**il s'agit d'un récepteur choisi parmi les récepteurs de glucocorticoïde, minéralcorticoïde, thyroïde, oestrogène, aldostérone et acide rétinoïque.

12. Vecteur viral selon l'une des revendications précédentes **caractérisé en ce que** la région d'encapsidation du virus est incluse dans la région d'ADN encadrée par les deux séquences permettant la recombinaison site-spécifique.

13. Vecteur viral selon l'une des revendications précédentes **caractérisé en ce qu'**il s'agit d'un adénovirus recombinant défectif.

14. Vecteur viral selon la revendication 13 **caractérisé en ce qu'**il s'agit d'un adénovirus comprenant une délétion de tout ou partie de la région E1.

15. Vecteur viral selon la revendication 14 **caractérisé en ce qu'**il s'agit d'un adénovirus comprenant en outre une délétion de tout ou partie de la région E4.

16. Vecteur viral selon l'une des revendications 1 à 12 **caractérisé en ce qu'**il s'agit d'un rétrovirus recombinant défectif.

17. Vecteur viral selon l'une des revendications 1 à 12 **caractérisé en ce qu'**il s'agit d'un AAV recombinant défectif.

18. Cellule modifiée par insertion d'un vecteur viral selon l'une des revendications 1 à 17.

19. Cellule selon la revendication 18, **caractérisée en ce qu'**il s'agit d'une cellule eucaryote.

20. Composition pharmaceutique comprenant au moins un vecteur viral selon l'une des revendications 1 à 17 ou une cellule selon la revendication 18.

21. Procédé de préparation in vitro de molécules d'ADN circulaires réplicatives **caractérisé en ce qu'**une population de cellules contenant un vecteur viral selon la revendication 3 est mise en présence de la recombinase permettant d'induire in situ la recombinaison site-spécifique.

22. Procédé selon la revendication 21 **caractérisé en ce que** la mise en présence avec la recombinase est effectuée par transfection ou infection desdites cellules avec un plasmide ou un vecteur viral contenant le gène de ladite recombinase.

23. Procédé de préparation in vitro de molécules d'ADN circulaires réplicatives comprenant :
(i) la transformation de cellules au moyen d'un vecteur viral comprenant :
- une région d'ADN délimitée par deux séquences permettant une recombinaison site-spécifique et positionnées en orientation directe, ladite région comprenant successivement un promoteur fonctionnel dans les cellules mammifères, la séquence oriP du virus EBV, une cassette d'expression comportant un gène d'intérêt et le gène de la protéine EBNA1 séparés par une séquence IRES dérivée du virus ECMV, le promoteur et la cassette d'expression étant orientés de telle sorte que l'expression des deux gènes n'est possible qu'après la recombinaison site-spécifique, et,
- le gène codant pour ladite recombinase sous contrôle d'un promoteur inductible, et,
(ii) l'induction de l'expression de la recombinase.

24. Utilisation d'un vecteur viral selon l'une quelconque des revendications 1 à 17 pour générer in vitro des molécules d'ADN circulaires réplicatives.

25. Vecteur viral selon l'une quelconque des revendications 1 à 17 en tant que médicament pour la thérapie génique.

26. Utilisation d'un vecteur viral selon l'une quelconque des revendications 1 à 17 pour la préparation d'un médicament pour la thérapie génique.

## Patentansprüche

1. Viraler Vektor, **dadurch gekennzeichnet, dass** er eine durch zwei eine situs-spezifische Rekombination erlaubende und in direkter Orientierung positionierte Sequenzen begrenzte DNA-Region umfasst, wobei die genannte Region sukzessive einen funktionalen Promoter in Säugetierzellen umfasst, die Sequenz oriP des Virus EBV, eine Expressionskassette mit dem Gen von Interesse und das Gen des Proteins EBNA1, die durch eine vom Virus ECMV abgeleitete Sequenz IHRES getrennt werden, wobei der Promoter und die Expressionskassette derart ausgerichtet sind, dass die Expression der beiden Gene erst nach der situs-spezifischen Rekombination möglich ist.

2. Viraler Vektor gemäß Anspruch 1, **dadurch gekennzeichnet, dass** die Expression des Gens von Interesse von der situs-spezifischen Rekombination abhängt.

3. Viraler Vektor gemäß Anspruch 1 und 2, **dadurch gekennzeichnet, dass** die die situs-spezifische Rekombination erlaubenden Sequenzen in Gegenwart einer Rekombinase zur spezifischen Rekombination fähigen Sequenzen sind.

4. Viraler Vektor gemäß Anspruch 3, **dadurch gekennzeichnet, dass** die die situs-spezifische Rekombination erlaubenden Sequenzen von einem Bakteriophagen abgeleitet sind.

5. Viraler Vektor gemäß Anspruch 4, **dadurch gekennzeichnet, dass** die die situs-spezifische Rekombination erlaubenden Sequenzen vom Bakteriophagen P1 abgeleitet sind.

6. Viraler Vektor gemäß Anspruch 5, **dadurch gekennzeichnet, dass** es sich um wiederholte umgekehrte Sequenzen des Bakteriophagen P1 handelt (Region loxP), dessen Rekombination durch die Rekombinase Cre induziert wird.

7. Viraler Vektor gemäß Anspruch 3, **dadurch gekennzeichnet, dass** er darüber hinaus neben der durch die beiden eine situs-spezifische Rekombination erlaubenden Sequenzen begrenzte Region das für die entsprechende Rekombinase kodierende Gen umfasst.

8. Viraler Vektor gemäß Anspruch 7, **dadurch gekennzeichnet, dass** das für die Rekombinase kodierende Gen unter die Kontrolle eines induktiven Promoters gebracht wird.

9. Viraler Vektor gemäß Anspruch 8, **dadurch gekennzeichnet, dass** der Promoter aus dem durch Dexamethason induktiven Promoter MMTV und einem durch Tetrazyklin induktiven Promoter ausgewählt wird.

10. Viraler Vektor gemäß Anspruch 8, **dadurch gekennzeichnet, dass** das Gen der Rekombinase darüber hinaus ein für den Verbindungsbereich eines Rezeptors eines Hormons kodierendes Regulationselement umfasst.

11. Viraler Vektor gemäß Anspruch 10, **dadurch gekennzeichnet, dass** es sich um einen aus den Glutokortikoid-, Mineralkortikoid-, Thyroid-, Östrogen-, Aldosteron- und Retinolsäure-Rezeptoren ausgewählten Rezeptor handelt.

12. Viraler Vektor gemäß einem der vorherigen Ansprüche, **dadurch gekennzeichnet, dass** die Kapsidierungs-Region des Virus in der durch die beiden, die situs-spezifische Rekombination erlaubenden Sequenzen umrahmten DNA-Region inbegriffen ist.

13. Viraler Vektor gemäß einem der vorherigen Ansprüche, **dadurch gekennzeichnet, dass** es sich um einen unvollständigen rekombinanten Adenovirus handelt.

14. Viraler Vektor gemäß Anspruch 13, **dadurch gekennzeichnet, dass** es sich um einen Adenovirus mit einer vollständigen oder partiellen Defizienenz der Region E1 handelt.

15. Viraler Vektor gemäß Anspruch 14, **dadurch gekennzeichnet, dass** es sich um einem Adenovirus handelt, der darüber hinaus eine vollständige oder partielle Defizienz der Region E4 aufweist.

16. Viraler Vektor gemäß Anspruch 1 bis 12, **dadurch gekennzeichnet, dass** es sich um einen unvollständigen rekombinanten Retrovirus handelt.

17. Viraler Vektor gemäß Anspruch 1 bis 12, **dadurch gekennzeichnet, dass** es um einen unvollständigen adeno-associated Virus handelt.

18. Durch Einfügen eines viralen Vektors gemäß Anspruch 1 bis 17 modifizierte Zelle.

19. Zelle gemäß Anspruch 18, **dadurch gekennzeichnet, dass** es sich um eine eukaryotische Zelle handelt.

20. Pharmazeutische Zusammensetzung mit wenigstens einem viralen Vektor gemäß Anspruch 1 bis 17 oder einer Zelle gemäß Anspruch 18.

21. Herstellungsverfahren in vitro von zirkulären, replikativen DNA-Molekülen, **dadurch gekennzeichnet, dass** eine einen viralen Vektor gemäß Anspruch 3 enthaltende Zellpopulation mit der die Induktion in situ der situs-spezifischen Rekombination erlaubenden Rekombinase in Kontakt gebracht wird.

22. Verfahren gemäß Anspruch 21, **dadurch gekennzeichnet, dass** der Kontakt mit der Rekombinase per Transfektion oder Infektion der genannten Zellen mit einem Plasmid oder einem das Gen der genannten Rekombinase enthaltenden viralen Vektor erfolgt.

23. Herstellungsverfahren in vitro von zirkulären, replikativen DNA-Molekühlen, umfassend:
(i) die Transformation von Zellen mittels eines viralen Vektors mit:
- einer durch zwei eine situs-spezifische Rekombination erlaubende und in direkter Ausrichtung positionierte Sequenzen begrenzten DNA-Region, wobei die genannte Region sukzessive einen funktionalen Promoter in Säugetierzellen, die Sequenz oriP des Virus EBV, eine Expressionskassette mit einem Gen von Interesse und dem Gen des Proteins EBNA1 umfasst, die durch eine vom Virus ECMV abgeleitete Sequenz IRES getrennt werden, wobei der Promoter und die Expressionskassette derart ausgerichtet sind, dass die Expression der beiden Gene nur nach der situs-spezifischen Rekombination möglich ist, und
- dem für die genannte Rekombinase unter der Kontrolle eines induktiven Promoters kodierenden Gen und
(ii) die Induktion der Expression der Rekombinase.

24. Einsatz eines viralen Vektors gemäß Anspruch 1 bis 17 zur Erzeugung von zirkulären, replikativen DNA-Molekülen in vitro.

25. Viraler Vektor gemäß Anspruch 1 bis 17 als Medikament zur Gentherapie.

26. Einsatz eines viralen Vektors gemäß Anspruch 1 bis 17 zur Herstellung eines Medikaments zur Gentherapie.

## Claims

1. Viral vector, **characterized in that** it comprises a DNA region delimited by two sequences which allow site-specific recombination and which are positioned in direct orientation, said region successively comprising a functional promoter in mammalian cells, the oriP sequence of the EBV virus, an expression cassette comprising a gene of interest and the gene of the EBNA1 protein separated by an IRES sequence derived from the ECMV virus, the promoter and the expression cassette being oriented such that expression of the two genes is possible only after site-specific recombination.

2. Viral vector according to Claim 1, **characterized in that** expression of the gene of interest is dependent on site-specific recombination.

3. Viral vector according to any one of Claims 1 and 2, **characterized in that** the sequences which allow site-specific recombination are sequences capable of recombining specifically in the presence of a recombinase.

4. Viral vector according to Claim 3, **characterized in that** the sequences which allow site-specific recombination are derived from a bacteriophage.

5. Viral vector according to Claim 4, **characterized in that** the sequences which allow site-specific recombination are derived from P1 bacteriophage.

6. Viral vector according to Claim 5, **characterized in that** these are inverted repeat sequences of P1 bacteriophage (loxP region), recombination of which is induced by Cre recombinase.

7. Viral vector according to Claim 3, **characterized in that** it also comprises, outside the region delimited by the two sequences which allow site-specific recombination, the gene which encodes the corresponding recombinase.

8. Viral vector according to Claim 7, **characterized in that** the gene which encodes the recombinase is placed under the control of an inducible promoter.

9. Viral vector according to Claim 8, **characterized in that** the promoter is selected from the MMTV promoter inducible by dexamethasone and a promoter inducible by tetracycline.

10. Viral vector according to Claim 8, **characterized in that** the gene of the recombinase also comprises a regulatory element which encodes the binding domain of a hormone receptor.

11. Viral vector according to Claim 10, **characterized in that** this is a receptor selected from glucocorticoid, mineralocorticoid, thyroid, oestrogen, aldosterone and retinoic acid receptors.

12. Viral vector according to any one of the preceding claims, **characterized in that** the virus encapsidation region is included in the DNA region delimited by the two sequences which allow site-specific recombination.

13. Viral vector according to any one of the preceding claims, **characterized in that** it is a defective recombinant adenovirus.

14. Viral vector according to Claim 13, **characterized in that** it is an adenovirus which comprises a deletion of all or part of the E1 region.

15. Viral vector according to Claim 14, **characterized in that** it is an adenovirus which also comprises a deletion of all or part of the E4 region.

16. Viral vector according to one of Claims 1 to 12, **characterized in that** it is a defective recombinant retrovirus.

17. Viral vector according to any one of Claims 1 to 12, **characterized in that** it is a defective recombinant AAV.

18. Cell which has been modified by insertion of a viral vector according to one of Claims 1 to 17.

19. Cell according to Claim 18, **characterized in that** it is a eukaryotic cell.

20. Pharmaceutical composition comprising at least one viral vector according to one of Claims 1 to 17 or a cell according to Claim 18.

21. Process for the in vitro preparation of circular replicative DNA molecules, **characterized in that** a population of cells containing a viral vector according to Claim 3 is brought into the presence of the recombinase which makes it possible to induce site-specific recombination in situ.

22. Process according to Claim 21, **characterized in that** the bringing into the presence of the recombinase is carried out by transfecting or infecting said cells with a plasmid or a viral vector containing the gene of said recombinase.

23. Process for the in vitro preparation of circular replicative DNA molecules, comprising:
(i) transforming cells by means of a viral vector comprising:
- a DNA region delimited by two sequences which allow site-specific recombination and which are positioned in direct orientation, said region successively comprising a functional promoter in mammalian cells, the oriP sequence of the EBV virus, an expression cassette comprising a gene of interest and the gene of the EBNA1 protein separated by an IRES sequence derived from the ECMV virus, the promoter and the expression cassette being oriented such that expression of the two genes is possible only after site-specific recombination, and
- the gene encoding said recombinase under the control of an inducible promoter, and
(ii)inducing the expression of the recombinase.

24. Use of a viral vector according to any one of Claims 1 to 17 for generating in vitro circular replicative DNA molecules.

25. Viral vector according to any one of Claims 1 to 17 as a medicament for gene therapy.

26. Use of a viral vector according to any one of Claims 1 to 17 for preparing a medicament for gene therapy.
